(19) Europäisches Patentamt
European Patent Office
Office européen des brevets

(11) **EP 3 128 895 B1**

(12) **FASCICULE DE BREVET EUROPEEN**

(45) Date de publication et mention de la délivrance du brevet:
**30.06.2021 Bulletin 2021/26**

(21) Numéro de dépôt: **15718543.0**

(22) Date de dépôt: **07.04.2015**

(51) Int Cl.:
*A61B 3/04* (2006.01)     *G02B 7/02* (2021.01)
*G02B 7/12* (2021.01)     *A61B 3/028* (2006.01)
*A61B 3/00* (2006.01)     *G02C 13/00* (2006.01)

(86) Numéro de dépôt international:
**PCT/FR2015/050890**

(87) Numéro de publication internationale:
**WO 2015/155456 (15.10.2015 Gazette 2015/41)**

(54) **LUNETTES DE COMPENSATION VISUELLE ET PROCEDE DE REFRACTION SUBJECTIVE D'UN INDIVIDU PORTANT CES LUNETTES**

KORRIGIERENDE BRILLE UND VERFAHREN ZUR SUBJEKTIVEN REFRAKTION DURCH EINEN TRÄGER DER BESAGTEN BRILLE

CORRECTIVE EYEGLASSES AND METHOD FOR SUBJECTIVE REFRACTION BY A WEARER OF SAID EYEGLASSES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.04.2014 FR 1453130**

(43) Date de publication de la demande:
**15.02.2017 Bulletin 2017/07**

(73) Titulaire: **Essilor International**
**94220 Charenton-le-Pont (FR)**

(72) Inventeurs:
• **BOUTINON, Stéphane**
  **F-94220 Charenton-le-Pont (FR)**
• **TEJEDOR DEL RIO, Vincent**
  **F-94220 Charenton-le-Pont (FR)**
• **NAUCHE, Michel**
  **F-94220 Charenton-le-Pont (FR)**

(74) Mandataire: **Jacobacci Coralis Harle**
**32, rue de l'Arcade**
**75008 Paris (FR)**

(56) Documents cités:
**EP-A2- 1 138 253     EP-A2- 1 250 883
EP-A2- 1 308 123     GB-A- 2 017 966
JP-A- 2000 002 857     JP-A- 2007 125 125
US-A- 3 880 502     US-A- 5 104 214
US-A1- 2003 174 282**

**Description**

DOMAINE TECHNIQUE AUQUEL SE RAPPORTE L'INVENTION

La présente invention concerne le domaine de l'optométrie.

Elle concerne plus particulièrement des lunettes de compensation visuelle, par exemple des lunettes d'essai, ainsi qu'un procédé de réfraction subjective d'un individu portant ces lunettes.

ARRIERE-PLAN TECHNOLOGIQUE

Dans le cadre de la mesure de l'acuité visuelle d'un patient, il a déjà été proposé de simuler la compensation visuelle à fournir, par exemple au moyen de lunettes d'essai ou d'un réfracteur, tel qu'une tête de réfraction.

Dans la tête de réfraction, des verres d'essai sont placés sur plusieurs disques, entraînés en rotation manuellement ou à l'aide d'un mécanisme motorisé. On comprend toutefois qu'un tel objet présente un encombrement et un poids importants, liés au nombre de verres placés sur chaque disque.

On connaît en particulier du document EP 1 308 123 un appareil d'optométrie comprenant deux chambres équipées chacune de disques rotatifs sur lesquels sont montés une pluralité d'éléments optiques dont l'un est placé au droit de l'oeil E d'un patient.

Les lunettes d'essais sont moins encombrantes. On prévoit en effet qu'elles accueillent successivement des verres d'essai ayant des corrections différentes, jusqu'à trouver la correction qui convient au patient.

Cette solution est toutefois assez peu pratique, notamment parce qu'elle nécessite le stockage séparé des verres d'essai dans des boîtes dédiées. Elle implique en outre des changements de lentilles qui provoquent des transitions de puissance de correction non désirées et non continues.

Le document US 2003/174 282 décrit en particulier un outil de test d'optométrie comprenant une monture conçue pour porter une lentille avec possibilité de rotation, et une pince pour fixation sur des lunettes.

De même, le document US 5 104 214 décrit des lunettes d'essais comprenant un ensemble de lentilles en face de chaque œil du patient, chaque ensemble de lentilles pouvant inclure deux lentilles cylindriques mobiles en rotation indépendamment l'une de l'autre.

On connaît par ailleurs du document EP 1 138 253 des lunettes d'essais comprenant des parties de détection d'information permettant de transmettre directement à un ordinateur des informations sur les propriétés de la réfraction effectuée.

OBJET DE L'INVENTION

Dans ce contexte, la présente invention propose des lunettes de compensation visuelle conformes à la revendication 1.

On peut ainsi obtenir une grande variété de corrections sur des lunettes : en effet, par la combinaison proposée des trois éléments optiques précités, on peut faire varier la puissance sphérique, la puissance cylindrique et l'angle de cylindre générés par le sous-ensemble optique.

Dans le mode de réalisation décrit, l'axe optique est perpendiculaire à l'axe de cylindre des premier et second éléments optiques et les premier et second éléments optiques n'exercent aucune puissance sphérique pour ladite direction de regard du porteur.

On prévoit par exemple que chacun des premier, second et troisième éléments optiques est une lentille de diamètre supérieur ou égal à 20 mm, ce qui permet d'obtenir un sous-ensemble optique ayant une taille suffisante pour placer facilement un œil en regard.

Le sous-ensemble optique comprend par exemple une carte électronique conçue pour commander la puissance sphérique du troisième élément optique, la position du premier élément optique autour de l'axe optique et la position du second élément optique en rotation autour de l'axe optique en fonction d'informations de consigne.

On peut prévoir par ailleurs que le sous-ensemble optique comprenne un inclinomètre et/ou un télémètre ; la carte électronique peut alors déterminer les informations de consigne en fonction notamment d'une information d'inclinaison reçue de l'inclinomètre et/ou du télémètre.

On peut également envisager d'utiliser un bouton actionnable par le porteur, de sorte que la carte électronique puisse modifier la puissance sphérique du troisième élément optique en cas d'appui sur le bouton.

On peut en outre prévoir un module de réception conçu pour recevoir les informations de consigne à travers une liaison sans fil. On évite ainsi la présence de fils qui gêneraient le porteur des lunettes. Celui-ci peut donc avoir une posture naturelle lorsqu'il porte les lunettes de compensation visuelle.

Les moyens de support comprennent par exemple un support nasal. Le sous-ensemble optique peut par ailleurs être monté sur un élément de monture, éventuellement de manière réglable selon un axe horizontal.

Le support nasal peut quant à lui être monté réglable sur l'élément de monture.

Les moyens de support peuvent en outre comprendre au moins une branche de longueur réglable.

Les lunettes peuvent également comprendre un système de stockage d'énergie (par exemple électrique) permettant d'alimenter (électriquement) des moyens conçus pour régler la puissance sphérique du troisième élément optique et/ou la position du premier élément optique autour de l'axe optique et/ou la position du second élément optique en rotation autour de l'axe optique, afin de rendre l'appareil autonome.

L'invention propose également un procédé de réfraction subjective d'un individu portant des lunettes comme proposées ci-dessus et comprenant les étapes suivantes :

- détermination d'un type de vision (vision de près, vision intermédiaire ou vision de loin) par utilisation de l'inclinomètre ou du télémètre ;
- détermination, par la carte électronique, d'au moins une information de consigne associée au type de vision déterminé ;
- ajustement de la puissance optique du troisième élément optique, de la position du premier élément optique ou de la position du second élément optique en fonction de l'information de consigne déterminée. Pour déterminer le type de vision, on utilise par exemple la distance de visée (distance de l'objet regardé selon la ligne de regard) déterminée grâce à l'inclinomètre ou grâce au télémètre ; des plages de valeurs de la distance de visée sont associées aux différents types de vision.

Un tel procédé peut également comprendre les étapes suivantes :

- détection d'un appui sur le bouton ;
- ajustement de la puissance optique en fonction des données reçues de la carte de commande.

DESCRIPTION DETAILLEE D'UN EXEMPLE DE RÉALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 représente schématiquement les éléments optiques utilisés dans un exemple de mise en œuvre de l'invention ;
- la figure 2 représente un vue en coupe d'un exemple de dispositif de compensation visuelle qui peut être utilisé dans le cadre de l'invention ;
- la figure 3 représente une vue écorchée du dispositif de compensation visuelle de la figure 2 côté lentilles cylindriques ;
- la figure 4 est une vue écorchée du dispositif de compensation visuelle de la figure 2 côté lentille sphérique variable ;
- la figure 5 représente schématiquement un élément de commande du dispositif de compensation visuelle de la figure 2 ;
- la figure 6 représente en vue de côté une paire de lunettes d'essai utilisant deux dispositifs de compensation visuelle du type de celui représenté sur les figures 2 à 4 ;
- la figure 7 représente en vue de face la paire de lunettes d'essai de la figure 6 ;
- la figure 8 présente un exemple classique d'utilisation des lunettes d'essai des figures 6 et 7.

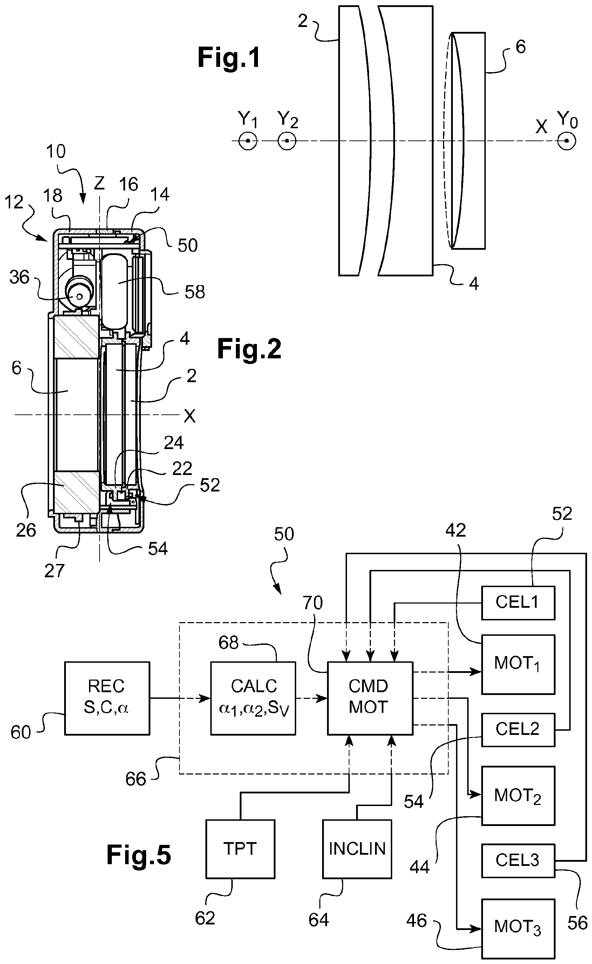

Sur la figure 1 sont schématiquement représentés les éléments optiques principaux d'un exemple de dispositif de compensation visuelle utilisé, comme décrit plus loin, dans des lunettes de compensation visuelle conformes aux enseignements de l'invention.

Ces éléments optiques comprennent une lentille plan-cylindre convexe 2, de puissance cylindrique $C_0$, une lentille plan-cylindre concave 4, de puissance cylindrique négative $-C_0$, et une lentille 6 de puissance sphérique variable $S_V$.

La valeur absolue (ou module), ici $C_0$, de la puissance cylindrique (ici $-C_0$) de la lentille plan-cylindre concave 4 est donc égale à la valeur absolue ($C_0$) (ou module) de la puissance cylindrique ($C_0$) de la lentille plan-cylindre convexe 2.

On pourrait prévoir en variante que les puissances cylindriques respectives de la lentille plan-cylindre concave 4 et de la lentille plan-cylindre convexe 2 soient (légèrement) différentes en valeur absolue, mais soient en tout état de cause telles que la puissance cylindrique résultante, générée par la combinaison de ces deux lentilles, ait une valeur négligeable (par exemple inférieur à 0,1 dioptrie en valeur absolue) dans au moins une position relative de ces deux lentilles.

Les trois lentilles 2, 4, 6 sont placées sur le même axe optique X. Précisément, chacune des trois lentilles 2, 4, 6 a une forme extérieure généralement cylindrique, centrée sur l'axe optique X. Dans l'exemple décrit ici, les lentilles 2, 4, 6 ont respectivement les diamètres (mesurant leur encombrement) suivants : 25 mm, 25 mm, 20 mm.

On remarque de ce fait qu'il est préférable d'utiliser ce dispositif de compensation visuelle 10 en positionnant l'œil du patient du côté de la lentille de puissance sphérique variable 6 de sorte que les lentilles de puissance cylindriques 2, 4, de plus grand diamètre, ne viennent pas limiter le champ de vision défini par la lentille de puissance sphérique variable 6, qui est lui-même large du fait de la proximité de l'œil du patient.

Chacune des trois lentilles 2, 4, 6 comporte une première face plane, perpendiculaire à l'axe optique X, et une seconde face, opposée à la première face et optiquement active :

- la face optiquement active de la lentille 2 est de forme cylindrique convexe (l'axe $Y_1$ du cylindre définissant cette face étant perpendiculaire à l'axe optique X) ;
- la face optiquement active de la lentille 4 est de forme cylindrique concave (l'axe $Y_2$ du cylindre définissant cette face étant perpendiculaire à l'axe optique X) ;
- la face optiquement active de la lentille 6 de puissance sphérique variable $S_V$ est déformable et peut ainsi prendre une forme sphérique convexe (comme illustré en pointillés sur la figure 1), une forme plane ou une forme sphérique concave (comme illustré en trait plein).

La lentille 6 de puissance sphérique variable $S_V$ est par exemple une lentille du type décrit dans le document EP 2 034 338. Une telle lentille comprend une cavité fermée par une membrane déformable transparente et une paroi plane transparente mobile ; la cavité contient un liquide transparent de volume constant qui est plus ou moins contraint par la face mobile, afin de déformer la membrane qui est de ce fait soit une surface concave sphérique, soit une surface plane, soit une surface convexe sphérique. Dans la lentille utilisée, une transformation de mouvement réalisée par un système vis écrou permet d'assurer la transformation de mouvement translation - rotation. Dans l'exemple décrit ici, la lentille 6 a une focale variable entre -40 mm et 40 mm, soit une puissance sphérique $S_V$ variable entre -25D et 25D (D étant la dioptrie, unité de mesure de la vergence, inverse de la focale exprimée en mètres).

Par ailleurs, les lentilles plan-cylindre 2, 4 ont respectivement comme déjà indiqué une puissance cylindrique $-C_0$ et $C_0$, ici avec $C_0 = 5D$.

Comme expliqué plus en détail dans la suite, la lentille plan-cylindre concave 4 et la lentille plan-cylindre convexe 2 sont montées en rotation autour de l'axe X (rotation centrée sur l'axe X).

L'axe $Y_1$ du cylindre convexe formé sur la face optiquement active de la lentille plan-cylindre convexe 2 peut ainsi former un angle variable $\alpha_1$ avec un axe de référence $Y_0$ (fixe et perpendiculaire à l'axe optique X).

De même, l'axe $Y_2$ du cylindre concave formé sur la face optiquement active de la lentille plan-cylindre concave 4 peut former un angle variable $\alpha_2$ avec l'axe de référence $Y_0$.

Par calcul de la vergence sur les différents méridiens, on obtient les formules suivantes pour la puissance sphérique S, la puissance cylindrique C et l'angle d'astigmatisme $\alpha$ du sous-ensemble optique formé des trois éléments optiques 2, 4, 6 qui vient d'être décrit :

$$\tan 2\alpha = \frac{\sin 2\alpha_2 - \sin 2\alpha_1}{\cos 2\alpha_2 - \cos 2\alpha_1} = -\frac{\cos\left(\alpha_1 + \alpha_2\right)}{\sin\left(\alpha_1 + \alpha_2\right)} \text{ (formule 1)}$$

$$C = C_0\left(\cos 2\left(\alpha - \alpha_2\right) - \cos 2\left(\alpha - \alpha_1\right)\right) \text{ (formule 2)}$$

$$S = S_V - \frac{C}{2} \text{ . (formule 3).}$$

On remarque que le terme (-C/2) dans la formule 3 correspond à une puissance sphérique générée par la résultante des 2 lentilles à puissance cylindrique.

En pilotant la position en rotation de la lentille plan-cylindre convexe 2 et la position en rotation de la lentille plan-cylindre concave 4, indépendamment l'une de l'autre, comme décrit ci-après, on peut faire varier indépendamment chacun des angles $\alpha_1$, $\alpha_2$ de 0° à 360° et ainsi obtenir une puissance cylindrique C réglable entre $-2.C_0$ et $2.C_0$ (soit ici entre -10D et 10D), et pour n'importe quel angle d'astigmatisme réglable entre 0° et 360° obtenue par une commande simultanée des deux lentilles. Comme l'indique la formule numéro 3, la résultante de puissance sphérique induite par la résultante de l'orientation des 2 lentilles cylindriques est compensée à l'aide de la lentille sphérique de puissance variable.

Par ailleurs, en faisant varier la puissance sphérique $S_V$ de la lentille sphérique 6, on peut régler la puissance sphérique S du sous-ensemble formé des trois lentilles 2, 4, 6.

Selon une variante envisageable, les lentilles à puissance cylindrique fixe pourraient avoir la même puissance cylindrique $C_0$ (positive ou négative) : il pourrait s'agir de deux lentilles plan-cylindre convexe, éventuellement identiques, ou, en alternative, de deux lentilles plan-cylindre concave, éventuellement identiques.

En effet, dans ce cas, la puissance sphérique S, la puissance cylindrique C et l'angle d'astigmatisme $\alpha$ du sous-ensemble formé de ces deux lentilles et d'une lentille à puissance sphérique variable sont donnés par les formules suivantes :

$$\tan 2\alpha = \frac{\sin 2\alpha_2 + \sin 2\alpha_1}{\cos 2\alpha_2 + \cos 2\alpha_1} \text{ (formule 4)}$$

$$C = C_0 \left(\cos 2(\alpha - \alpha_2) + \cos 2(\alpha - \alpha_1)\right) \text{ (formule 5)}$$

$$S = S_V + C_0 - \frac{C}{2} . \text{ (formule 6)}$$

Le terme $C_0$ - C/2 correspond à la puissance sphérique induite par la combinaison des deux lentilles à puissance cylindrique.

On peut donc également dans ce cas régler la puissance sphérique S, la puissance cylindrique C et l'angle d'astigmatisme $\alpha$, en particulier de sorte que la puissance cylindrique C soit nulle, en entraînant en rotation les lentilles à puissance cylindrique (indépendamment l'une de l'autre) et en faisant varier la puissance sphérique de la lentille à puissance sphérique variable.

Un exemple de dispositif de compensation visuelle 10 qui utilise les éléments optiques qui viennent d'être décrits est représenté en figure 2.

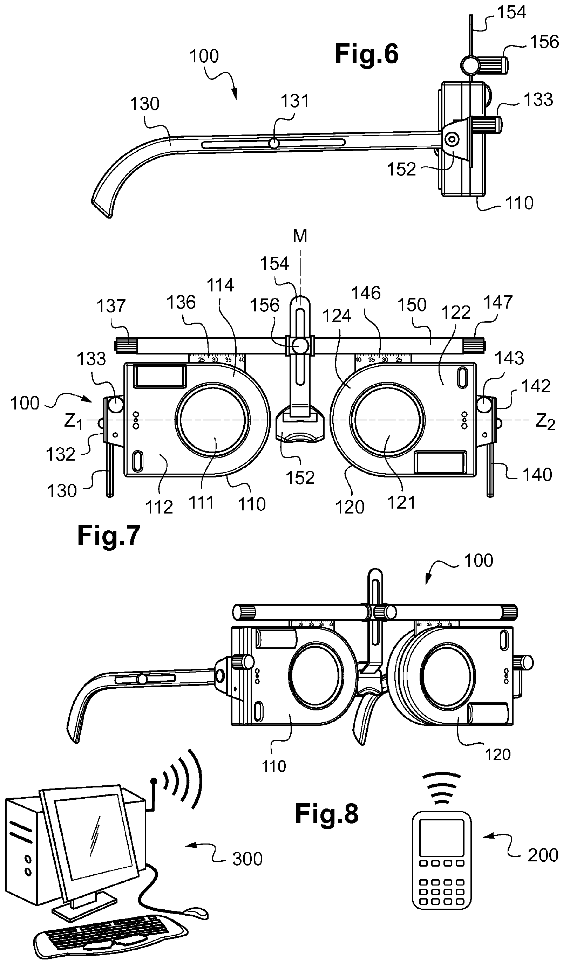

On utilisera parfois dans la description qui suit, afin de clarifier l'explication, des termes, comme "*supérieur*" ou "*inférieur*", qui définissent une orientation dans les figures 2, 3 et 4. On comprend que cette orientation n'est pas nécessairement applicable à l'utilisation qui pourra être faite du dispositif décrit, en particulier celle des figures 6 à 8.

Le dispositif de compensation visuelle 10 comprend un boîtier 12 formé d'une première partie 14, d'une seconde partie 16 et d'une troisième partie 18, qui s'étendent successivement selon l'axe optique X et sont assemblées deux à deux au niveau de plans perpendiculaires à l'axe optique X.

Une première roue dentée 22 est montée en rotation centrée sur l'axe optique X dans la première partie 14 du boîtier 12 et porte en son centre, dans une ouverture prévue à cet effet, la lentille plan-cylindre convexe 2. La première roue dentée 22 et la lentille plan-cylindre convexe 2 sont coaxiales ; autrement dit, en section dans un plan perpendiculaire à l'axe optique X, la circonférence extérieure de la première roue dentée 22 et la circonférence de la lentille plan-cylindre convexe 2 forment des cercles concentriques centrés sur l'axe optique X.

De même, une seconde roue dentée 24 est montée en rotation centrée sur l'axe optique X dans la seconde partie 16 du boîtier 12 et porte en son centre, dans une ouverture prévue à cet effet, la lentille plan-cylindre concave 4. La seconde roue dentée 24 et la lentille plan-cylindre concave 4 sont coaxiales ; autrement dit, en section dans un plan perpendiculaire à l'axe optique X, la circonférence extérieure de la seconde roue dentée 24 et la circonférence de la lentille plan-cylindre concave 4 forment des cercles concentriques centrés sur l'axe optique X.

Une troisième roue dentée 27 est montée en rotation centrée sur l'axe optique X dans la troisième partie 18 du boîtier 12. La troisième roue dentée 27 est solidaire d'une bague pourvue sur la circonférence d'un boîtier 26 qui porte la lentille 6 de puissance sphérique variable et permettant la commande de la puissance sphérique $S_V$. Le boîtier 26 de la lentille 6 de puissance sphérique variable est monté dans la troisième partie 18 du boîtier 12.

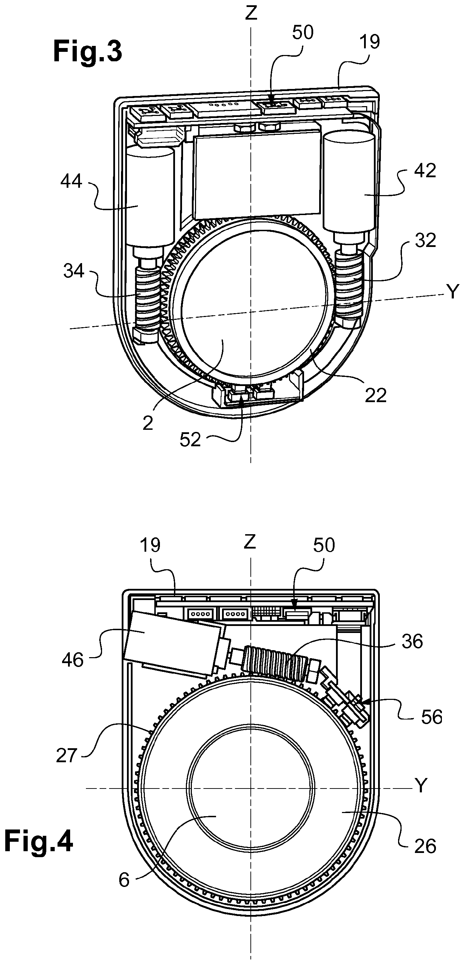

Comme bien visible en figure 3, la première roue dentée 22 est entraînée en rotation (autour de l'axe optique X) au moyen d'un premier moteur 42 dont un axe d'entraînement porte une première vis sans fin 32 qui engrène sur la première roue dentée 22. Le premier moteur 42 est par exemple monté dans la première partie 14 du boîtier 12.

La position courante de la première roue dentée 22 est surveillée par une première cellule optique 52.

De même, la seconde roue dentée 24 est entraînée en rotation autour de l'axe optique X au moyen d'un second moteur 44 dont un axe d'entraînement porte une seconde vis sans fin 34 qui engrène sur la seconde roue dentée 24. Le second moteur 44 est par exemple monté dans la seconde partie 16 du boîtier 12.

La position courante de la seconde roue dentée 24 est surveillée par une seconde cellule optique 54.

Comme représenté sur la figure 4, la troisième roue dentée 27 est quant à elle entraînée en rotation (autour de l'axe optique X) au moyen d'un troisième moteur 46 qui présente un axe d'entraînement sur lequel est monté une troisième vis sans fin 36 qui engrène avec la troisième roue dentée 27. Le troisième moteur 46 est par exemple monté dans la troisième partie 18 du boîtier 12.

La position courante de la troisième roue dentée 27 est surveillée par une troisième cellule optique 56.

Les premier, second et troisième moteurs 42, 44, 46 sont par exemple des moteurs pas à pas, d'une résolution de 20 pas/tour, pilotés ici en 8ème de pas (ci-après micro-pas). En variante, ces moteurs pourraient être pilotés en 16ème de pas.

Le volume interne du boîtier 12 (comme d'ailleurs le volume interne de chacune des première, seconde et troisième parties 14, 16, 18 de la même manière) peut être subdivisé en un espace de réception des moteurs 42, 44, 46 (région supérieure du boîtier 12 sur les figures 2, 3 et 4) et un espace de réception des éléments optiques 2, 4, 6 (région inférieure du boîtier 12 sur les figures 2, 3 et 4).

L'espace de réception des moteurs 42, 44, 46 a une forme essentiellement parallélépipédique, ouverte (vers le bas sur les figures) en direction de l'espace de réception des éléments optiques 2, 4, 6 et fermé à l'opposé (vers le haut sur les figures) par une face supérieure 19 du boîtier 12 (la face supérieure 19 du boîtier 12 étant formée par l'assemblage de faces supérieures respectives des première, seconde et troisième parties 14, 16, 18 du boîtier 12).

La disposition des moteurs 42 44 et 46 est telle qu'elle permet de bénéficier d'une géométrie circulaire sur 180° centrée sur l'axe optique au plus proche du rayon utile des lentilles.

L'espace de réception des éléments optiques 2, 4, 6 présente, à l'opposé de l'espace de réception des moteurs, une forme cylindrique (délimitée par les parois du boîtier 12) qui épouse celle de la troisième roue dentée 27 sur la moitié de la circonférence de celle-ci.

Autrement dit, le boîtier 12 (et par conséquent chacune des première, seconde et troisième parties 14, 16, 18 du boîtier 12) a, au niveau de l'espace de réception des éléments optiques 2, 4, 6, une forme cylindrique de diamètre (perpendiculairement à l'axe optique X) du même ordre que, et légèrement supérieur à, celui de la troisième roue dentée 27.

Les diamètres respectifs des roues dentées 22, 24, 27 sont adaptés de manière à favoriser la conservation du champ en dépit de l'épaisseur du sous-ensemble optique.

Le premier moteur 42 et la première vis sans fin 32 s'étendent dans le boîtier 12 selon une direction Z perpendiculaire à la face supérieure du boîtier 12 (et donc notamment perpendiculaire à l'axe optique X) de telle sorte que le premier moteur 42 est logé dans l'espace de réception des moteurs tandis que la première vis sans fin 32 s'étend dans l'espace de réception des éléments optiques.

Le second moteur 44 et la seconde vis sans fin 34 s'étendent quant à eux dans le boîtier 12 selon la même direction, mais à l'opposé du premier moteur 42 et de la première vis sans fin 34 par rapport aux lentilles de puissance cylindrique 2, 4. Le second moteur 44 est logé dans l'espace de réception des moteurs tandis que la seconde vis sans fin 34 s'étend dans l'espace de réception des éléments optiques.

On remarque qu'ainsi la première vis sans fin 32 et la seconde vis sans fin 34 sont situées de part et d'autre de l'ensemble formé par la première roue dentée 22 et la seconde roue dentée 24, et que l'encombrement latéral (selon un axe Y perpendiculaire aux axes X et Z précités) de ces différentes pièces (première vis sans fin 32, seconde vis sans fin 34, première ou seconde roue dentée 22, 24) est inférieur au diamètre de la troisième roue dentée 27 de sorte que les première et seconde vis sans fin 32, 34 contiennent dans l'espace de réception des éléments optiques sans nécessiter d'excroissance pour les accueillir.

Par ailleurs, les premier et second moteurs 42, 44 ont chacun un encombrement selon l'axe optique X supérieur à celui de chacune des première et seconde roues dentées 22, 24, et même supérieur à celui de chacune des première et seconde parties 14, 16 de boîtier 12. Toutefois, du fait que ces premier et second moteurs 42, 44 sont placés comme il vient d'être indiqué de chaque côté du boîtier 12 (par rapport à l'axe Z), ils peuvent chacun occuper un espace qui s'étend selon l'axe optique X au droit de la première partie 14 et de la seconde partie 16 du boîtier 12.

Par exemple, chacun des premier et second moteurs 42, 44 a un encombrement latéral (diamètre externe du moteur) compris entre 6 et 12, par exemple 10 mm, tandis que les première et seconde roues dentées 22, 24 ont chacune une épaisseur (encombrement selon l'axe X) compris entre 1 et 4, par exemple 2,5 mm.

Le troisième moteur 46 et la troisième vis sans fin 36 sont en revanche situés dans l'espace de réception des moteurs, dans la région qui s'étend selon l'axe X au droit de la troisième partie 18 du boîtier 12. Ainsi, la troisième vis sans fin 36 engrène la troisième roue dentée 27 dans une partie supérieure de celle-ci, ce qui permet au boîtier 12 d'épouser la forme du boîtier 12 dans la partie inférieure de la troisième roue dentée 27, comme déjà indiqué.

Dans l'exemple décrit, comme visible en figure 4, l'axe du troisième moteur 46 et de la troisième vis sans fin 36 est légèrement incliné par rapport à la face supérieure du boîtier 12 (précisément par rapport à l'axe Y précité).

On prévoit par exemple que l'épaisseur de la troisième roue dentée 27 est comprise entre 0,3 mm et 2 mm.

Cette disposition des différents éléments permet d'obtenir un boîtier relativement fin, ayant typiquement une épaisseur comprise entre 15 et 20 mm.

Le boîtier 12 comprend également, par exemple dans la région supérieure de l'espace de réception des moteurs, un élément de commande 50, formé ici de plusieurs circuits intégrés portés par un circuit imprimé commun.

Par ailleurs un dispositif de stockage d'énergie électrique de type batterie 58 (ou, en variante, une super capacité) est prévu pour rendre l'appareil autonome. On prévoit par exemple également des éléments de recharge sans contact du dispositif de stockage d'énergie 58. La batterie 58 permet notamment l'alimentation électrique des moteurs 42, 44, 46 et de l'élément de commande 50.

A ce titre, les éléments de commande et de contrôle seront sélectionnés préférentiellement pour leur faible consommation.

Les éléments principaux d'un tel élément de commande 50, ainsi que leur connexion aux moteurs 42, 44, 46 précités et aux cellules optiques 52, 54, 56 précitées, sont représentés schématiquement en figure 5.

L'élément de commande 50 comprend un module de réception 60 conçu pour recevoir, ici à travers une liaison sans fil, les informations de consigne, c'est-à-dire des informations indicatives des valeurs souhaitées par l'utilisateur pour la puissance sphérique S, la puissance cylindrique C et l'angle d'astigmatisme $\alpha$ qui définissent la compensation générée par le sous-ensemble optique formé des éléments optiques 2, 4, 6.

Le module de réception 60 est par exemple un module de réception infrarouge qui reçoit ces informations de consigne d'une télécommande à émission infrarouge manipulée par l'utilisateur. En variante, on pourrait prévoir que ces informations de consigne soit reçues d'un ordinateur personnel via une liaison sans fil, par exemple un réseau local sans fil ; l'utilisateur pourrait dans ce cas choisir des valeurs de puissance sphérique S, de puissance cylindrique C et d'angle d'astigmatisme $\alpha$ pour le dispositif de compensation visuelle par sélection interactive sur l'ordinateur.

Le module de réception 60 transmet les informations de consigne S, C, $\alpha$ reçues à un calculateur 66 (constitué par exemple d'un processeur exécutant un programme d'ordinateur de manière à mettre en œuvre les fonctions du calculateur décrites ci-après), précisément à un module de calcul 68 mis en œuvre par ce calculateur 66.

Le module de calcul 68 calcule les valeurs des angles $\alpha_1$, $\alpha_2$ et la valeur de puissance sphérique $S_V$ nécessaires afin d'obtenir les valeurs de consignes S, C, $\alpha$ reçues en entrée, sur la base des formules exposées plus haut. Dans le cas où les lentilles plan-cylindre 2 et 4 ont respectivement une puissance cylindrique - $C_0$ et $C_0$, on utilise par exemple les formules suivantes :

$$\begin{cases} \alpha_1 = \alpha - \dfrac{1}{2}\arcsin\left(\dfrac{C}{2C_0}\right) + \dfrac{\pi}{4} \\[2mm] \alpha_2 = \alpha + \dfrac{1}{2}\arcsin\left(\dfrac{C}{2C_0}\right) + \dfrac{\pi}{4} \end{cases}$$

$$S_V = S + \frac{C}{2}$$

Le calculateur 66 met également en œuvre un module de commande 70 qui reçoit en entrée les valeurs d'angle $\alpha_1$, $\alpha_2$ et de puissance sphérique $S_V$ calculées par le module de calcul 68 et émet des signaux de commande à destination des moteurs 42, 44, 46 afin de commander chacun des moteurs 42, 44, 46 indépendamment des autres de manière à obtenir des positionnements respectifs des roues dentées 22, 24, 27 qui permettent d'obtenir les valeurs souhaitées :

- le module de commande 70 commande le premier moteur 42 de manière à faire tourner la première roue dentée 22 autour de l'axe optique X jusqu'à la position où l'axe $Y_1$ de la surface cylindrique optiquement active de la lentille plan-cylindre convexe 2 (portée par la première roue dentée 22) forme un angle $\alpha_1$ avec la direction de référence $Y_0$ ;
- le module de commande 70 commande le second moteur 44 de manière à faire tourner la seconde roue dentée 24 autour de l'axe optique X jusqu'à la position où l'axe $Y_2$ de la surface cylindrique optiquement active de la lentille plan-cylindre concave 4 (portée par la seconde roue dentée 24) forme un angle $\alpha_2$ avec la direction de référence $Y_0$ ;
- le module de commande 70 commande le troisième moteur 46 de manière à faire tourner la troisième roue dentée 27 autour de l'axe optique X jusqu'à la position où la bague de commande de la puissance sphérique variable commande la puissance sphérique $S_V$ calculée par le module de calcul 68.

La position de chaque roue dentée 22, 24, 27 est connue à chaque instant respectivement grâce aux cellules optiques 52, 54, 56 qui mesurent chacune, sur la roue dentée à laquelle chacune est associée, le nombre de dents ayant traversé la cellule optique par rapport à un point de référence sur la circonférence de la roue concernée (par exemple dépourvu de dent).

Dans l'exemple décrit ici, l'ensemble premier moteur 42-première vis sans fin 32-première roue dentée 22, comme l'ensemble second moteur 44-seconde vis sans fin 34-seconde roue dentée 24, génère une démultiplication telle qu'un tour de roue dentée 22, 24 correspond à 15040 micro-pas du moteur associé 42, 44. La résolution (angle de rotation des roues dentées 22, 24 pour un micro-pas) est donc de 0,024° pour les angles $\alpha_1$ et $\alpha_2$.

L'ensemble troisième moteur 46-troisième vis sans fin 36-troisième roue dentée 46 génère quant à lui une démultiplication de 16640 micro-pas par tour. La bague de commande de la puissance sphérique variable est réglable sur une plage angulaire de 120° (ce qui correspond donc à 5547 micro-pas) afin d'obtenir la variation de puissance sphérique de -25D à 25D (soit une plage de variation de 50D). La résolution (variation de puissance sphérique $S_V$ pour un micro-pas) est donc de 0,009D.

On peut prévoir que, lors du passage de valeurs de consigne initiales $\alpha_1$, $\alpha_2$, $S_V$ à de nouvelles valeurs de consigne $\alpha'_1$, $\alpha'_2$, $S'_V$, chacun des premier, second et troisième moteurs 42, 44, 46 soient actionnés pendant une même durée T (en secondes), qui peut dépendre éventuellement de l'amplitude de l'un des changements de consigne (par exemple de la variation, en valeur absolue, de puissance sphérique $| S'_V - S_V |$, où $| x |$ est la valeur absolue de x).

Pour ce faire, le calculateur 66 détermine par exemple le nombre $p_1$ de micro-pas du moteur 42 permettant le passage de l'angle $\alpha_1$ à l'angle $\alpha'_1$, le nombre $p_2$ de micro-pas du moteur 44 permettant le passage de l'angle $\alpha_2$ à l'angle $\alpha'_2$ et le nombre $p_3$ de micro-pas du moteur 46 permettant le passage de la puissance sphérique $S_V$ à la puissance sphérique $S'_V$. Le calculateur 66 commande alors la rotation du moteur 42 à une vitesse de $p_1/T$ micro-pas par seconde, la rotation du moteur 44 à une vitesse de $p_2/T$ micro-pas par seconde et la rotation du moteur 46 à une vitesse de $p_3/T$ micro-pas par seconde.

L'élément de commande 50 comprend également un capteur de température 62, qui délivre une information de température ambiante mesurée, et un inclinomètre 64, par exemple réalisé sous forme d'un accéléromètre et qui délivre une information d'orientation du dispositif de compensation visuelle 10, par exemple par rapport à la verticale. Dans l'application décrite ci-après en référence aux figures 6 à 8, l'information d'orientation peut être utilisée afin de déterminer la position du dispositif de compensation visuelle, et par conséquent quel œil est corrigé par ce dispositif de compensation visuelle, et/ou l'inclinaison du dispositif de compensation visuelle par rapport à la verticale afin de déterminer si l'utilisateur regarde en vision de loin, en vision intermédiaire ou en vision de près.

Le calculateur 66 reçoit l'information de température en provenance du capteur de température 62 et l'information d'orientation en provenance de l'inclinomètre 64 et utilise l'une au moins de ces informations dans le cadre de la détermination des commandes à envoyer aux moteurs 42, 44, 46.

Dans l'exemple décrit, le module de commande 70 utilise l'information de température afin de compenser les variations de puissance sphérique de la lentille 6 dues à la température (qui sont de l'ordre de 0,06D/°C dans l'exemple décrit) et l'information d'orientation afin de compenser les perturbations éventuelles du système d'entraînement (moteurs, vis sans fin, roues dentées) dues à des changements d'orientation du dispositif de compensation visuelle 10.

Contrairement au cas de la description des figures 2 à 4 ci-dessus, la description qui suit des figures 6 et 7 fait référence à des directions (horizontale et verticale notamment) et à des positionnements relatifs ("*inférieur*" ou "*supérieur*") qui correspondent à l'utilisation des lunettes de compensation visuelle (ici des lunettes d'essai) pour la mesure d'acuité visuelle du porteur.

Les figures 6 et 7 représentent, respectivement en vue de côté et en vue de face, une paire de lunettes d'essai utilisant deux dispositifs de compensation visuelle 110, 120 du type qui vient d'être décrit en référence aux figures 1 à 5.

Les deux dispositifs de compensation visuelle 110, 120 sont ici identiques mais montés sur des moyens de support sur le visage d'un porteur, comme expliqué plus en détail ci-dessous, de façon à être disposés de manière symétrique par rapport à un plan vertical médian M qui correspond au plan sagittal du porteur.

Précisément, le dispositif de compensation visuelle 110 destiné à l'œil droit du porteur est disposé tel que son espace (ici parallélépipédique) de réception des moteurs 112 est situé latéralement et vers l'extérieur (c'est-à-dire à droite vu du porteur) de son espace (ici cylindrique) de réception des éléments optiques 114 (c'est-à-dire de l'oculaire 111 du dispositif de compensation visuelle 110).

Autrement dit, l'axe $Z_1$ du dispositif de compensation visuelle 110 (qui correspond à l'axe Z des figures 2 à 4 pour le dispositif de compensation visuelle 110) est perpendiculaire au plan médian M (plan sagittal du porteur) et l'espace de réception des éléments optiques 114 (ou l'oculaire 111) est situé entre l'espace de réception des moteurs 112 et le plan médian M.

De même, le dispositif de compensation visuelle 120 destiné à l'œil gauche du porteur est disposé tel que son espace (ici parallélépipédique) de réception des moteurs 122 est situé latéralement et vers l'extérieur (c'est-à-dire à gauche vu du porteur) de son espace (ici cylindrique) de réception des éléments optiques 124 (c'est-à-dire de l'oculaire 121 du dispositif de compensation visuelle 120).

Autrement dit, l'axe $Z_2$ du dispositif de compensation visuelle 120 (qui correspond à l'axe Z des figures 2 à 4 pour le dispositif de compensation visuelle 120) est perpendiculaire au plan médian M (plan sagittal du porteur) et l'espace de réception des éléments optiques 124 (ou l'oculaire 121) est situé entre l'espace de réception des moteurs 122 et le plan médian M.

La paire de lunettes d'essai 100 comprend deux branches 130, 140 montées respectivement sur le dispositif de compensation visuelle 110 et sur le dispositif de compensation visuelle 120, chaque fois sur une face d'extrémité latérale du dispositif de compensation visuelle 110, 120 concerné et au moyen d'une attache latérale 132, 142.

Chaque branche 130, 140 comprend une partie coudée (de positionnement de l'oreille du porteur) à son extrémité opposée au dispositif de compensation visuelle 110, 120 concerné. Chaque branche 130, 140 est en outre réglable en longueur au moyen d'un système de réglage adapté 131 (par exemple une possibilité de coulissement entre deux demi-branches formant la branche 130, 140 concernée) afin de pouvoir ajuster la distance entre les yeux du patient et les dispositifs de compensation visuelle 110, 120.

Chaque branche 130, 140 est montée sur l'attache latérale correspondante 132, 142 avec une possibilité de réglage en rotation autour d'un axe horizontal (parallèle à l'axe $Z_1$, $Z_2$ défini ci-dessus) par exemple au moyen d'une molette 133, 143 afin de pouvoir ajuster l'angle pantoscopique.

Comme déjà indiqué, l'attache latérale 132, 142 est fixée sur la paroi d'extrémité latérale (référencée 19 sur les figures 3 et 4) du dispositif de compensation visuelle concerné 110, 120 (c'est-à-dire, pour l'attache latérale 132, à droite du dispositif de compensation visuelle 110 destiné à l'œil droit du porteur et, pour l'attache latérale 142, à gauche du dispositif de compensation visuelle 120 destiné à l'œil gauche du porteur). De chaque côté, l'espace de réception des moteurs 112, 122 est donc situé entre l'attache latérale 132, 142 et l'espace de réception des éléments optiques 114, 124 (ou l'oculaire 111, 121).

Les dispositifs de compensation visuelle 110, 120 sont tous deux montés sur une traverse 150 formant élément de monture, de part et d'autre du plan médian M, respectivement au moyen d'un premier coulisseau 136 et d'un second coulisseau 146.

La position de chacun des premier et second coulisseaux 136, 146 est réglable en translation selon la direction d'extension de la traverse 150 (par exemple au moyen d'une molette 137, 147 prévue à cet effet), ce qui permet un réglage selon une direction horizontale perpendiculaire au plan médian M (c'est-à-dire au plan sagittal du porteur) de la position de chaque dispositif de compensation visuelle 110, 120. On peut ainsi adapter (indépendamment l'une de l'autre) les positions respectives des dispositifs de compensation visuelle 110, 120 aux demi-distances pupillaires côté droit et côté gauche du porteur.

Un support nasal 152 (conçu pour venir en appui sur la partie supérieure du nez du porteur) est monté sur la traverse 150, au milieu de celle-ci (c'est-à-dire au niveau du plan médian M), par l'intermédiaire d'une attache centrale 154 pourvue d'une ouverture oblongue qui reçoit un pion solidaire de la traverse 150 afin de permettre un réglage selon une direction verticale de la position relative du support nasal 152 et de la traverse 150. Ce réglage s'effectue par exemple au moyen d'une molette 156 prévue à cet effet.

On peut prévoir en outre une possibilité de rotation de l'attache centrale 154 autour de l'axe horizontal d'extension de la traverse 150 afin de régler la position du support nasal 152 en profondeur (c'est-à-dire selon l'axe optique des dispositifs de compensation visuelle 110, 120).

On décrit à présent en référence à la figure 8 un exemple classique d'utilisation des lunettes d'essai 100 qui viennent d'être décrites.

Les lunettes d'essai 100 sont placées sur le visage du patient, en ajustant les différents réglages décrits ci-dessus à la morphologie du patient, dans les règles de l'art.

L'examen visuel peut alors commencer.

Le praticien envoie des consignes (informations indicatives des valeurs souhaitées par l'utilisateur pour la puissance sphérique S, la puissance cylindrique C et l'angle d'astigmatisme $\alpha$) pour l'œil droit et pour l'œil gauche au moyen de la liaison sans fil mentionnée ci-dessus.

Pour ce faire, il utilise par exemple comme déjà indiqué une télécommande infrarouge 200 conçue pour envoyer des données représentatives des consignes aux éléments de commande 50 via les modules de réception 60 implantés respectivement dans le dispositif de compensation visuelle 110 et dans le dispositif de compensation visuelle 120. Comme déjà indiqué, le praticien pourrait utiliser en variante un ordinateur 300, conçu par exemple pour établir un réseau local sans fil (ou "*Wireless Local Area Network*") avec les modules de réception 60 (qui sont dans cette variante des modules de réception radio).

On peut également prévoir que les données représentatives des consignes soient émises par un appareil électronique ayant effectué une mesure d'amétropie du patient. Les lunettes d'essai 100 seront dans ce cas utilisées pour valider la réfraction résultant des mesures d'amétropie.

On propose par exemple comme déjà indiqué que le dispositif électronique du praticien (télécommande, ordinateur ou appareil de mesure d'amétropie dans les exemples qui viennent d'être évoqués) émettent des données représentatives des consignes pour les deux yeux et que l'élément de commande 50 embarqué dans chaque dispositif de compensation visuelle 110, 120 détermine quelles consignes lui sont destinées.

Pour ce faire, on prévoit ici que l'élément de commande 50 détermine, sur la base des informations d'orientation reçues de l'accéléromètre 64, quelle est l'orientation du dispositif de compensation visuelle 110, 120 concerné et par conséquent à quel œil ce dispositif de compensation visuelle 110, 120 est associé. En effet, dans l'exemple décrit, les dispositifs de compensation visuelle 110, 120 sont identiques et sont montés de manière symétrique par rapport au plan médian M, comme déjà indiqué.

En variante, on pourrait prévoir que l'élément de commande 50 mémorise une information indicative de la position (à droite ou à gauche) du dispositif de compensation visuelle 110, 120 concerné dans la paire de lunettes d'essai 100.

On peut prévoir en outre que, pour chaque œil et pour chaque paramètre (puissance sphérique S, puissance cylindrique C et angle d'astigmatisme $\alpha$), plusieurs valeurs de consigne soient transmises au dispositif de compensation visuelle 110, 120 concerné, les différentes valeurs étant associées à différents angles d'inclinaison du dispositif concerné par rapport à la verticale, ou à différentes plages angulaires d'inclinaison du dispositif concerné par rapport à la verticale, ou encore à différents types de vision (vision de loin, vision intermédiaire, vision de près).

Pour un dispositif de compensation visuelle, on entend par inclinaison par rapport à la verticale (dans le cadre de lunettes de compensation visuelle telles que celles des figues 6 et 7) l'angle formé par l'axe Y des figures 3 et 4 avec la verticale, qui correspond à l'inclinaison par rapport à l'horizontale de l'axe optique X du dispositif de compensation visuelle.

Lorsque le porteur se tient tête droite et regarde au loin (vision de loin) cet angle est nul ou faible (inférieur à 10°) ; en revanche, en vision de près, cet angle est classiquement de 30°.

Lorsqu'un dispositif de compensation visuelle 110, 120 (et précisément son élément de commande 50) reçoit différentes valeurs de consigne (associées à différentes valeurs d'inclinaison) pour un paramètre, il détermine au moyen des informations d'orientation reçues de l'accéléromètre 64 l'inclinaison courante par rapport à la verticale et commande les éléments optiques (comme expliqué ci-dessus en référence à la figure 5) en utilisant les valeurs des paramètres associés à l'inclinaison ainsi déterminée.

Concernant la vision intermédiaire, on peut prévoir que le dispositif électronique du praticien (télécommande, ordinateur) émette des valeurs spécifiques des différents paramètres pour une plage d'inclinaison comprenant l'inclinaison de 20° (classiquement associée à la vision intermédiaire), par exemple la plage de valeurs comprises entre 15° et 25 °. En variante, l'élément de commande 50 pourrait calculer des valeurs des différents paramètres pour la vision intermédiaire à partir de valeurs correspondantes reçues pour la vision de loin et pour la vision de près, et appliquer ces valeurs calculées lorsqu'il détermine, sur la base des informations d'orientation reçues de l'accéléromètre 64, que l'inclinaison courante est comprise dans la plage précitée.

Selon une possibilité de réalisation, on peut prévoir, par exemple dans chaque dispositif de compensation visuelle 110, 120, un télémètre conçu pour mesurer la distance de l'objet observé dans la direction de regard (par exemple au moyen d'un système à ultrasons ou par triangulation). L'élément de commande 50 peut alors adapter la puissance sphérique en fonction de la distance de l'objet observé, par exemple en augmentant la puissance sphérique lorsque l'objet est proche afin de compenser un défaut d'accommodation visuelle.

Le produit est un équipement d'optique, sans fragilité particulière mais compte tenu de sa portabilité, on peut prévoir une base pour assurer la recharge sans contact des deux dispositifs de compensation visuelle 110, 120 et/ou vérifier sa calibration en utilisant un instrument de type frontofocomètre, afin de garantir le niveau de résultat souhaité.

On comprend de la description qui précède que les lunettes d'essai décrites ci-dessus pourront être utilisées à des fins de réfraction subjective, que ce soit en vision de loin, en vision intermédiaire ou en vision de près.

Ces lunettes d'essai présentent par rapport à des lunettes d'essai traditionnelles le bénéfice d'une très grande réactivité, de ne pas avoir à retirer les lunettes de la tête du porteur pour en modifier la correction, et d'offrir une valeur de correction évolutive en fonction de l'inclinaison de la tête.

On peut également prévoir d'utiliser de telles lunettes dans des conditions de champ de vision maîtrisées, à partir d'un écran présentant des stimuli mobiles ; les accéléromètres équipant les dispositifs de compensation visuelle vont alors permettre d'enregistrer les mouvements de la tête du patient nécessaires au suivi de la cible.

On pourra donc par soustraction des mouvements de la tête par rapport au mouvement théorique qu'aurait dû provoquer la cible en déduire le mouvement intrinsèque des yeux.

Afin de réaliser une réfraction monoculaire, un dispositif occultant non représenté pourra être placé sur la fenêtre optique de l'un des oculaires 111, 121. En alternative, l'automatisation du dispositif permettra de réaliser cette séparation droite/gauche permettant la réfraction monoculaire au moyen d'un brouillage sur l'œil à occulter par ajout d'une puissance optique prédéterminée (par exemple d'une valeur d'une dioptrie environ).

Selon une autre utilisation envisageable, les lunettes de compensation visuelle proposées ci-dessus pourront être utilisées en tant que montage test permettant par exemple de reproduire la future correction dans les conditions d'utilisations réelles, par exemple pour une démonstration d'utilisation de verres progressifs mi-distance.

Dans ce contexte, on peut prévoir en outre un bouton supplémentaire sur une face extérieure des dispositifs de compensation visuelle 110, 120 qui permette, lorsqu'il est enfoncé, une modification prédéterminée de la valeur d'un paramètre de correction (puissance sphérique S, puissance cylindrique C ou angle d'astigmatisme $\alpha$).

Le porteur des lunettes d'essai peut ainsi obtenir (par exemple au moyen de plusieurs pressions sur le bouton) un réglage qui lui convient mieux.

# EP 3 128 895 B1

**Revendications**

1. Lunettes de compensation visuelle comprenant des moyens de support (130, 140, 152) sur un visage d'un porteur et au moins un sous-ensemble optique (110, 120) monté sur les moyens de support (130, 140, 152) en regard d'au moins un des yeux du porteur,
le sous-ensemble optique (110, 120) comprenant trois éléments optiques montés sur les moyens de support en série suivant un axe optique (X), dont un premier élément optique (2) de puissance cylindrique pour une direction de regard du porteur suivant l'axe optique (X), un second élément optique (4) de puissance cylindrique pour ladite direction de regard et un troisième élément optique (6) de puissance sphérique variable pour ladite direction de regard, le premier élément optique (2) et le second élément optique (4) étant réglables en rotation autour de l'axe optique (X) indépendamment l'un de l'autre, une roue dentée (27) étant montée en rotation sur l'axe optique et étant solidaire d'une bague pourvue sur la circonférence d'un boîtier qui porte le troisième élément optique (6) et permettant la commande de ladite puissance sphérique.

2. Lunettes selon la revendication 1, dans lesquelles l'axe optique (X) est perpendiculaire à l'axe de cylindre des premier et second éléments optiques.

3. Lunettes selon la revendication 1 ou 2, dans lesquelles les premier et second éléments optiques n'exercent aucune puissance sphérique pour ladite direction de regard du porteur.

4. Lunettes selon l'une des revendications 1 à 3, dans lesquelles chacun des premier, second et troisième éléments optiques (2, 4, 6) est une lentille de diamètre supérieur ou égal à 20 mm.

5. Lunettes selon l'une des revendications 1 à 4, dans lesquelles le sous-ensemble optique (110, 120) comprend une carte électronique (50) conçue pour commander la puissance sphérique du troisième élément optique (6), la position du premier élément optique (2) autour de l'axe optique (X) et la position du second élément optique (4) en rotation autour de l'axe optique (X) en fonction d'informations de consigne.

6. Lunettes selon la revendication 5, dans lesquelles le sous-ensemble optique comprend un inclinomètre (64) et dans lesquelles la carte électronique (50) est conçue pour déterminer les informations de consigne en fonction d'une information d'inclinaison reçue de l'inclinomètre (64).

7. Lunettes selon la revendication 5, dans lesquelles le sous-ensemble optique comprend un télémètre et dans lesquelles la carte électronique (50) est conçue pour déterminer les informations de consigne en fonction d'une information de distance reçue du télémètre.

8. Lunettes selon l'une des revendications 5 à 7, comprenant un bouton actionnable par le porteur, dans lesquelles la carte électronique est conçue pour modifier la puissance sphérique du troisième élément optique en cas d'appui sur le bouton.

9. Lunettes selon la revendication 5, comprenant un module de réception conçu pour recevoir les informations de consigne à travers une liaison sans fil.

10. Lunettes selon l'une des revendications 1 à 9, dans lesquelles les moyens de support comprennent un support nasal (152).

11. Lunettes selon l'une des revendications 1 à 10, dans lesquelles le sous-ensemble optique (110, 120) est monté sur un élément de monture (150).

12. Lunettes selon la revendication 11, dans lesquelles le sous-ensemble optique (110, 120) est monté réglable selon un axe horizontal sur l'élément de monture (150).

13. Lunettes selon l'une des revendications 1 à 12 disposant d'un système de stockage d'énergie permettant d'alimenter des moyens conçus pour régler la puissance sphérique du troisième élément optique (6), la position du premier élément optique (2) autour de l'axe optique (X) et la position du second élément optique (4) en rotation autour de l'axe optique (X).

14. Procédé de réfraction subjective d'un individu portant des lunettes selon la revendication 6 ou 7, comprenant les étapes suivantes :

- détermination d'un type de vision par utilisation de l'inclinomètre ou du télémètre ;
- détermination, par la carte électronique (50), d'au moins une information de consigne associée au type de vision déterminé ;
- ajustement de la puissance optique du troisième élément optique (6), de la position du premier élément optique (2) ou de la position du second élément optique (4) en fonction de l'information de consigne déterminée.

15. Procédé de réfraction subjective d'un individu portant des lunettes selon la revendication 8, comprenant les étapes suivantes :

- détection d'un appui sur le bouton ;
- ajustement de la puissance optique en fonction des données reçues de la carte de commande.

**Patentansprüche**

1. Sehausgleichsbrille umfassend Mittel zum Halt (130, 140, 152) auf einem Gesicht eines Trägers und mindestens eine optische Untereinheit (110, 120), die auf den Haltemitteln (130, 140, 152) gegenüber mindestens eines der Augen des Trägers angebracht ist, wobei die optische Untereinheit (110, 120) drei optische Elemente umfasst, die an den Haltemitteln in Reihe entlang einer optischen Achse (X) angebracht sind, davon ein erstes optisches Element (2) zylindrischer Kraft für eine Blickrichtung des Trägers entlang der optischen Achse (X), ein zweites optisches Element (4) zylindrischer Kraft für die Blickrichtung und ein drittes optisches Element (6) variabler sphärischer Kraft für die Blickrichtung, wobei das erste optische Element (2) und das zweite optische Element (4) um die optische Achse (X) unabhängig voneinander drehverstellbar sind, wobei ein Zahnrad (27) auf der optischen Achse drehbar gelagert ist und mit einem Ring fest verbunden ist, der auf dem Umfang eines Gehäuses vorgesehen ist, das das dritte optische Element (6) trägt und die Steuerung der sphärischen Kraft gestattet.

2. Brille nach Anspruch 1, wobei die optische Achse (X) senkrecht zur Zylinderachse des ersten und zweiten optischen Elements verläuft.

3. Brille nach Anspruch 1 oder 2, wobei das erste und zweite optische Element keine sphärische Kraft für die Blickrichtung des Trägers ausüben.

4. Brille nach einem der Ansprüche 1 bis 3, wobei jedes des ersten, zweiten und dritten optischen Elements (2, 4, 6) eine Linse mit einem Durchmesser größer als oder gleich 20 mm ist.

5. Brille nach einem der Ansprüche 1 bis 4, wobei die optische Untereinheit (110, 120) eine Platine (50) umfasst, die dafür ausgelegt ist, die sphärische Kraft des dritten optischen Elements (6), die Position des ersten optischen Elements (2) um die optische Achse (X) und die Position des zweiten optischen Elements (4) in Drehung um die optische Achse (X) in Abhängigkeit von Vorgabeinformationen zu steuern.

6. Brille nach Anspruch 5, wobei die optische Untereinheit einen Neigungsmesser (64) umfasst, und wobei die Platine (50) dafür ausgelegt ist, die Vorgabeinformationen in Abhängigkeit von einer Neigungsinformation zu bestimmen, die vom Neigungsmesser (64) empfangen wird.

7. Brille nach Anspruch 5, wobei die optische Untereinheit einen Abstandsmesser umfasst, und wobei die Platine (50) dafür ausgelegt ist, die Vorgabeinformationen in Abhängigkeit von einer Abstandsinformation zu bestimmen, die vom Abstandsmesser empfangen wird.

8. Brille nach einem der Ansprüche 5 bis 7, umfassend einen Knopf, der vom Träger betätigbar ist, wobei die Platine dafür ausgelegt ist, die sphärische Kraft des dritten optischen Elements im Fall des Drucks auf den Knopf zu ändern.

9. Brille nach Anspruch 5, umfassend ein Empfangsmodul, das dafür ausgelegt ist, die Vorgabeinformationen über eine Drahtlosverbindung zu empfangen.

10. Brille nach einem der Ansprüche 1 bis 9, wobei die Haltemittel einen Nasenhalter (152) umfassen.

**11.** Brille nach einem der Ansprüche 1 bis 10, wobei die optische Untereinheit (110, 120) auf einem Gestellelement (150) angebracht ist.

**12.** Brille nach Anspruch 11, wobei die optische Untereinheit (110, 120) entlang einer horizontalen Achse verstellbar auf dem Gestellelement (150) angebracht ist.

**13.** Brille nach einem der Ansprüche 1 bis 12, die über ein Energiespeichersystem verfügt, das es gestattet, Mittel zu versorgen, die dafür ausgelegt sind, die sphärische Kraft des dritten optischen Elements (6), die Position des ersten optischen Elements (2) um die optische Achse (X) und die Position des zweiten optischen Elements (4) in Drehung um die optische Achse (X) zu verstellen.

**14.** Verfahren zur subjektiven Refraktion einer Person, die eine Brille nach Anspruch 6 oder 7 trägt, umfassend die folgenden Schritte:

- Bestimmen eines Sehtyps unter Verwendung des Neigungsmessers oder des Abstandsmessers;
- Bestimmen, durch die Platine (50), mindestens einer Vorgabeinformation, die dem bestimmten Sehtyp zugeordnet ist;
- Einstellen der optischen Kraft des dritten optischen Elements (6), der Position des ersten optischen Elements (2) oder der Position des zweiten optischen Elements (4) in Abhängigkeit von der bestimmten Vorgabeinformation.

**15.** Verfahren zur subjektiven Refraktion einer Person, die eine Brille nach Anspruch 8 trägt, umfassend die folgenden Schritte:

- Detektieren eines Drucks auf den Knopf;
- Einstellen der optischen Kraft in Abhängigkeit von den Daten, die von der Steuerkarte empfangen werden.

**Claims**

**1.** Vision compensating spectacles comprising means of support (130, 140, 152) on a wearer's face and at least one optical subassembly (110, 120) mounted on the support means (130, 140, 152) opposite at least one of the wearer's eyes,
the optical subassembly (110, 120) comprising three optical elements mounted on the support means in series along an optical axis (X), including a first optical element (2) of cylindrical power for a direction of gaze of the wearer along the optical axis (X), a second optical element (4) of cylindrical power for said direction of gaze and a third optical element (6) of variable spherical power for said direction of gaze, the first optical element (2) and the second optical element (4) being adjustable in rotation about the optical axis (X) independently of one another, a toothed wheel (27) being mounted in rotation about the optical axis and being secured to a ring provided on the circumference of a casing which carries the third optical element (6) and allowing control of said spherical power.

**2.** Spectacles according to Claim 1, wherein the optical axis (X) is perpendicular to the cylinder axis of the first and second optical elements.

**3.** Spectacles according to Claim 1 or 2, wherein the first and second optical elements do not exert any spherical power for the said direction of gaze of the wearer.

**4.** Spectacles according to one of Claims 1 to 3, wherein each of the first, second and third optical elements (2, 4, 6) is a lens of diameter greater than or equal to 20 mm.

**5.** Spectacles according to one of Claims 1 to 4, wherein the optical subassembly (110, 120) comprises an electronic card (50) designed to control the spherical power of the third optical element (6), the position of the first optical element (2) about the optical axis (X) and the position of the second optical element (4) in rotation about the optical axis (X) as a function of setpoint information.

**6.** Spectacles according to Claim 5, wherein the optical subassembly comprises an inclinometer (64) and wherein the electronic card (50) is designed to determine the setpoint information as a function of an item of inclination information received from the inclinometer (64).

**EP 3 128 895 B1**

7. Spectacles according to Claim 5, wherein the optical subassembly comprises a telemeter and wherein the electronic card (50) is designed to determine the setpoint information as a function of an item of distance information received from the telemeter.

8. Spectacles according to one of Claims 5 to 7, comprising a button actuatable by the wearer, wherein the electronic card is designed to modify the spherical power of the third optical element should the button be pressed.

9. Spectacles according to Claim 5, comprising a reception module designed to receive the setpoint information through a wireless link.

10. Spectacles according to one of Claims 1 to 9, wherein the support means comprise a nasal support (152).

11. Spectacles according to one of Claims 1 to 10, wherein the optical subassembly (110, 120) is mounted on a frame element (150).

12. Spectacles according to Claim 11, wherein the optical subassembly (110, 120) is mounted adjustable along a horizontal axis on the frame element (150).

13. Spectacles according to one of Claims 1 to 12 employing an energy storage system making it possible to energize means designed to adjust the spherical power of the third optical element (6), the position of the first optical element (2) about the optical axis (X) and the position of the second optical element (4) in rotation about the optical axis (X).

14. Method of subjective refraction of an individual wearing spectacles according to Claim 6 or 7, comprising the following steps:

- determination of a type of vision by use of the inclinometer or the telemeter;
- determination, by the electronic card (50), of at least one item of setpoint information associated with the determined type of vision;
- adjustment of the optical power of the third optical element (6), of the position of the first optical element (2) or of the position of the second optical element (4) as a function of the determined item of setpoint information.

15. Method of subjective refraction of an individual wearing spectacles according to Claim 8, comprising the following steps:

- detection of the pressing of the button;
- adjustment of the optical power as a function of the data received from the control card.

14

## Fig.1

2

6

$Y_1$  $Y_2$  X  $Y_0$

4

## Fig.2

10

12  18  Z  16  14

50

36

58

6

4

2

X

24

22  52

26

27  54

## Fig.5

50

70

68

42  52

CEL1

REC
S,C,α

CALC
$\alpha_1, \alpha_2, S_V$

CMD
MOT

MOT₁

60

CEL2

66

54

MOT₂

TPT

INCLIN

44

CEL3

62

64

46

MOT₃

56

EP 3 128 895 B1

**Fig.3**

**Fig.4**

Fig.6

Fig.7

Fig.8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1308123 A
- US 2003174282 A
- US 5104214 A
- EP 1138253 A
- EP 2034338 A